# EUROPÄISCHE PATENTANMELDUNG

(11) **EP 3 375 453 A1**
(43) Veröffentlichungstag der Anmeldung: **19.09.2018**
(21) Anmeldenummer: 17160651.0
(22) Anmeldetag: 13.03.2017
(51) Int. Cl.: A61K 38/47, C12N 9/28, A23K 10/10, A23K 20/189, A23K 50/10

(54) **VERWENDUNG EINER PANSENGESCHÜTZEN ALPHA-AMYLASE**

(71) Anmelder: BASF SE, 67056 Ludwigshafen am Rhein (DE)
(72) Erfinder: TROESCHER, Arnulf, 68623 Lampertheim (DE); MUESCHEN, Hans, 68623 Lampertheim (DE); FEUERSTEIN, Dieter, 68623 Lampertheim (DE); ADER, Peter, 68623 Lampertheim (DE)
(74) Vertreter: BASF IP Association

(57) **Zusammenfassung**

Verwendung einer pansengeschützen Amylase zur Senkung der Milchsäure Ausscheidung im Kot bei Wiederkäuern, welche mit stärkereichen Rationen gefüttert werden.

## Beschreibung

Die vorliegende Erfindung betrifft die Verwendung von alpha-Amylasen, die gegenüber den Bedingungen des Pansen geschützt sind, bei der Ernährung von Wiederkäuern, die mit stärkereichen Rationen ernährt werden.

Bei der Ernährung von Wiederkäuern werden statt des natürlichen Futters in Form von überwiegend Gras auf der Weide oder Heu im Winter im Stall insbesondere in den Hochleistungsbetrieben immer höhere Anteile an sogenanntem Kraftfutter verfüttert, um den Tieren mehr Energie zuzuführen und die Erträge zu steigern. Diese Hochleistungsfutter enthalten einen hohen Anteil an Getreide bzw. Stärke und entsprechen damit nicht der natürlichen, dem Verdauungssystem von Wiederkäuern entsprechenden, Futterzusammensetzung. Bekannt ist hier das Auftreten einer Pansenübersäuerung (Pansenacidose) und der damit verbundenen Schädigung der komplexen Pansenflora, insbesondere bei plötzlicher Futterumstellung von Gras auf stärkereiche Futter bzw. zu hohen Getreideanteilen, die durch das überdurchschnittliche Wachstum von stärkespaltenden Lactobazillen in einem stärkereichen Umfeld und der von den Lactobazillen produzierten Milchsäure verursacht wird. Die produzierte überdurchschnittliche Menge an Milchsäure senkt den pH vom normalen neutralen ins stark saure ab und führt zu einer Schädigung der Pansenschleimhaut und Absterben der physiologischen Mikroorganismen im Pansen, wobei schwere Acidosen bis zum Tot der Tiere führen können, da die natürliche Abpufferung bzw. Neutralisierung der Milchsäure im Pansen durch die im Speichel der Wiederkäuer enthaltenen Bicarbonate nicht mehr ausreichend möglich ist. Zur Behandlung ist hier kurzfristig die zusätzliche Verabreichung von Natrium-Bicarbonat im Futter möglich.

Vernachlässigt wurde bisher die analoge Milchsäureproduktion durch Lactobazillen im Darm bedingt durch die aus dem Pansen austretende unverdaute Stärke. Dies kann auf hohe Stärkemengen und/oder die Verfütterung von pansenstabiler Stärke zurückgeführt werden. Nach 6h unter Pansenbedingungen ist bei stärkereichen Rationen noch ein erheblicher Teil der mit dem Futter aufgenommenen Stärke unverdaut und gelangt dann aus dem Pansen in das anschliessende Verdauungssystem. Im Darm, der normalerweise auch einen neutralen pH aufweist, wird durch die immer noch vorhandene Stärke ebenfalls das Wachstum von Lactobazillen überproportional gefördert und die Milchsäureproduktion derart gesteigert, dass es analog der Übersäuerung des Pansens zu einer Übersäuerung des Darms kommt. Da hier weder durch körpereigenen abpuffernden Speichel noch durch Zufütterung von Bicarbonaten eine Neutralisierung des sauren pH stattfinden kann, reagiert der Körper einerseits mit der Zuführung von Wasser aus dem Körper in den Darm zum Verdünnen und Ausschwemmen des unerwünschten, sauren Darminhalts, was sich in massiven anhaltenden Durchfällen der betroffenen Tieren äussert, und andererseits wird die Darmschleimhaut durch den sauren pH entzündet, geschädigt und abgestossen, was zu schwerwiegenden Erkrankungen, Blutungen bis zum Tot der Tieren führen kann. Gleichzeitig ist Stärke, die in den Dickdarm gelangt, energetisch vom Tier nur noch teilweise zu verwerten. Die daraus gebildeten kurzkettigen Fettsäuren können zwar noch absorbiert werden, nicht aber das mikrobielle synthetisierte Protein.

Aufgabe der Erfindung war es, die Übersäuerung des Darms bzw. Zerstörung der Darmschleimhaut durch die im Darm produzierte Milchsäure bei Wiederkäuern zu reduzieren bzw. verhindern, die mit stärkereichen Rationen ernährt werden.

Diese Aufgabe wird gelöst durch die Verwendung einer pansengeschützen exogenen alpha-Amylase zur Senkung der Milchsäure Ausscheidung im Kot von Wiederkäuern, welche mit stärkereichen Rationen mit einem Stärkeanteil von mindestens 25 Gew% bezogen auf die Gesamtfuttertrockenmasse gefüttert werden. Die Amylase, die durch den Pansenschutz nicht selbst bereits im Pansen von dort vorhanden Mikroorganismen, insbesondere von deren Proteasen, abgebaut wird, kann einen Abbau der Stärke im Dünndarm bewirken, sodass keine Substrate mehr für die Lactobazillen im Dickdarm vorhanden sind und entsprechend keine Milchsäure gebildet und pH und Darmschleimhaut normal bzw. intakt bleiben. Milchsäure im Sinne der Erfindung bedeutet messbare Gesamtmilchsäure in Form von D- und L- Milchsäure im Kot.

Lediglich die Verfütterung von Amylasen bei stärkereduzierten Rationen und Auswirkungen auf die Milchproduktion und die aufgenommene Futtermenge bei Milchkühen wurde von Gencoglu et al. untersucht (J. Dairy Sci. 93: 723-732). Hierbei wurde eine ungeschützte, im Pansen aktive, Amylase verfüttert, die den Stoffwechsel im Pansen beeinflusst.

Unter pansengeschützter Amylase im Sinne der Erfindung ist eine derart formulierte Amylase gemeint, die eine verzögerte Freisetzung der Amylase, vorzugsweise durch den sauren pH im Labmagen, aus der Formulierung erst nach Passage des Pansens aufweist. Vorzugsweise beträgt die maximale Freisetzung nach 6h unter Pansenbedingungen 25% des Enzyms. Vorzugsweise werden 50-100% der anfänglich vorhandenen Amylase innerhalb von 6-24h im an den Pansen anschliessenden Verdauungstrakt freigesetzt.

Der Schutz gegen den Abbau von Futtermittelzusätzen im Pansen ist bereits seit langem bekannt und dem Fachmann sind diverse Arten und Technologien geläufig wie sich alle Arten von Wirkstoffen wie bspw. Aminosäuren, Vitamine, Pigmente oder Enzyme vor einem Abbau bzw. einer Freisetzung im Pansen schützen lassen. Diese werden beschrieben unter anderem in der FR 2401620, FR 2401621, FR 2514261, EP406041, EP427639 und EP 1360904.

Vorzugsweise ist die pansengeschützte Amylase pelletierstabil, entweder durch die Eigenschaften des Proteins selbst oder durch die Art der Formulierung, d.h. sie erleidet keine signifikanten Aktivitätsverluste unter Pelletierbedingungen wie heissem Dampf von 70 bis 100 °C, Druck und Scherkräften, wie sie bei der Herstellung von Futtermittelpellets auftreten. Dies ermöglicht die Einarbeitung der pansengeschützen alpha-Amylase in übliche Futtermittelpellets und damit eine leichtere Dosierung im Stall bei der Verfütterung.

Es ist auch denkbar, dass eine alpha-Amylase eingesetzt wird, die durch molekularbiologische Verfahren so verändert wurde, dass sie nicht mehr durch die im Pansen vorhanden Mikroorganismen abgebaut werden kann.

In einer besonderen Ausführungsform beträgt der Stärkeanteil bezogen auf die Gesamtfuttertrockenmasse mindestens 30, 35, 40, 45, 50, 55, 60, 65, 70, 75 oder 80 Gew.%, je nachdem, um welche Wiederkäuer es sich handelt und in welchem Produktions- oder Laktationsabschnitt sich die Tiere befinden.

Es ist auch denkbar, dass besondere Körner, Getreide oder Futterprodukte enthaltend pansenstabile Stärken verfüttert werden, um einen Abbau unter Pansenbedingungen und eine Pansenacidose zu verhindern. Im Fall von pansenstabilen Stärken kommt daher die gleiche Menge unverdaute Stärke aus weniger aufgenommener Stärke bezogen auf die Gesamtfuttertrockenmasse aus dem Pansen im folgenden Verdauungssystem an. Entsprechend reduziert sich der Stärkeanteil gemäss der Erfindung je stabiler die Stärke gegenüber dem Pansenabbau ist. Im Fall einer Pansenstabilität von mindestens 50% gegenüber einer normalen Stärke reduziert sich der Stärkeanteil der stärkereichen Ration gemäss der vorliegenden Erfindung ebenfalls um entsprechend 50 % auf mindestens 12,5 %. Daher beträgt bei einer Pansenstabilität der Stärke von mindestens 50% der Stärkeanteil mindestens 12,5 Gew%, insbesondere mindestens 15, 20, 25, 30, 35 oder 40 Gew.%. Im Fall einer Pansenstabilität von mindestens 25% gegenüber einer normalen Stärke reduziert sich der Stärkeanteil der stärkereichen Ration gemäss der vorliegenden Erfindung ebenfalls um entsprechend 25 %. Daher beträgt bei einer Pansenstabilität der Stärke von mindestens 25% der Stärkeanteil mindestens 19 Gew%, insbesondere mindestens 20, 25, 30, 35 oder 40 Gew.%.

Die Menge an Stärke, die ausgehend vom Stärkeanteil im Futtermittel den Pansen passiert und im Darm zur Milchsäure umgesetzt werden kann, variiert je nach Art des Getreides in dem die Stärke vorhanden ist, da manche Getreidestärken leichter im Pansen von den Mikroorganismen abbaubar sind, andere aufgrund von sekundären Getreidebestandteilen wie bspw. des Zeins im Fall von Mais, die einen natürlichen Pansenschutz darstellen, deutlich schwerer abbaubar sind. So wird im Fall von Mais die darin enthaltene und verfütterte Stärke nur zu 50% im Pansen abgebaut, sodass von 2 kg verfütterter Stärke noch 1 kg den Pansen passiert, im Fall von Gerste wird die verfütterte Stärke zu 75 %, im Fall von Weizen zu 85% im Pansen abgebaut, sodass von 4kg bzw. 6,7 kg verfütterter Stärke nur noch 1 kg nach dem Pansen vorhanden sind. Entsprechend ist die Menge, d.h. Units, an Amylase, die dem Futter zugesetzt wird, abhängig von der Art des Getreides bzw. der Getreidestärke. Unter Stärke im Futter wird vorliegend der Anteil an Stärke im verfütterten Getreide oder der Silage verstanden, die an die Tiere verfüttert werden.

In einer Ausführungsform werden daher mindestens 26000, vorzugsweise mindestens 30000, insbesondere mindestens 40000. Units Amylase pro kg nach dem Pansen verfügbare Stärke eingesetzt.

In einer anderen Ausführungsform werden mindestens 4000, vorzugsweise mindestens 6500, insbesondere mindestens 13000, Units Amylase pro kg Stärke im Futter eingesetzt, je nach Art der Stärke und deren Abbaugrad im Pansen.

Vorzugsweise werden im Fall von Weizen bzw. auf Weizen basierendem Futter mindestens 4000, vorzugsweise mindestens 5000, insbesondere mindestens 6000, Units Amylase pro kg Stärke im Futter eingesetzt.

Vorzugsweise werden im Fall von Gerste bzw. auf Gerste basierendem Futter mindestens 6500, vorzugsweise mindestens 8000, insbesondere mindestens10000, Units Amylase pro kg Stärke im Futter eingesetzt.

Vorzugsweise werden im Fall von Mais bzw. auf Mais basierendem Futter mindestens 13000, vorzugsweise mindestens 17000, insbesondere mindestens 20000, Units Amylase pro kg Stärke im Futter eingesetzt.

Im Fall von selten vorkommenden aber denkbaren gemischten Getreiderationen, werden die entsprechenden Getreidebestandteile und deren Stärkeanteile entsprechend anteilsmässig bei der Berechnung der mindestens einzusetzen Menge an Amylase berücksichtigt.

Vorteilhafterweise wird durch die erfindungsgemässe Verwendung die mittlere Milchsäure Ausscheidung im Kot pro Tag gegenüber der gleichen stärkereichen Ration ohne eine exogene alpha-Amylase um mindestens 40%, vorzugsweise mind. 50%, insbesondere mind. 60, 70, 80 oder 90 %, gesenkt. Damit wird eine Absenkung des pH im Dickdarm und Entzündung der Darmschleimhaut vermieden bzw. das Risiko der Entzündung deutlich gesenkt. Die Höhe der Absenkung ist hierbei abhängig vom Stärkeanteil in der Ration. So ist bei höheren Stärkeanteilen eine entsprechend höhere Absenkung notwendig, um die absolute Milchsäuremenge, die pro Tag produziert und ausgeschieden wird, auf einem Darm- bzw. Darmschleimhaut verträglichen Niveau zu halten. Unter der mittleren Milchsäureausscheidung pro Tag wird gemäss der vorliegenden Erfindung der Mittelwert der täglichen Ausscheidungen im Kot aus 5 aufeinanderfolgenden Tagen verstanden.

Vorzugsweise sind die Wiederkäuer ausgewählt aus der Gruppe bestehend aus Rindern, insbesondere Milchkühen oder Mastrindern, Schafen und Ziegen.

Die Erfindung umfasst weiterhin die Verwendung einer pansengeschützten exogenen alpha-Amylase zur Erhöhung der post-Pansen Stärkeverwertung bei Wiederkäuern, welche mit stärkereichen Rationen mit einem Stärkeanteil von mindestens 25 Gew.% bezogen auf die Gesamtfuttertrockenmasse gefüttert werden.

Indem die Amylase, die nach dem Pansen, insbesondere im Dünndarm, die den Pansen passierte Stärke abbaut und diverse Zucker, insbesondere Maltose und/oder Glucose, als Abbauprodukte bereitstellt, die wiederum im Dünndarm aufgenommen und dem Energiehaushalt des Wiederkäuers zu Gute kommen, erhöht sie die pro kg verfütterter Stärke im Wiederkäuer gewonnen Energie und damit die Stärkeverwertung. Diese exogene Amylase bewirkt, dass post ruminale Stärke im Dünndarm abgebaut und bereits dort in Form von Glukose absorbiert wird. Dies ermöglicht eine höhere energetische Verwertung von Glucose im Tier.

In einer besonderen Ausführungsform beträgt der Stärkeanteil bezogen auf die Gesamtfuttertrockenmasse mindestens 30, 35, 40, 45, 50, 55, 60, 65, 70, 75 oder 80 Gew.%, je nachdem, um welche Wiederkäuer es sich handelt und in welchem Produktions- oder Laktationsabschnitt sich die Tiere befinden.

Die Erfindung umfasst weiterhin die Bereitstellung einer pansengeschützten exogenen alpha-Amylase zur Behandlung einer stärkebedingten Dickdarm Acidose bei Wiederkäuern. Da eine Abpufferung des Dickdarms im Fall einer aufgetretenen Übersäuerung nicht analog der Abpufferung des Pansens durch Zusatz eines Bicarbonats zum Futter möglich ist, stellt der Einsatz einer pansenstabilen Amylase eine Möglichkeit dar, um eine Normalisierung des pH durch durch das Verhindern der Milchsäureproduktion und Ausscheidung an Milchsäure im Kot und, nach Abklingen des Durchfalls, eine Darmschleimhaut Regeneration zu ermöglichen.

Die Erfindung umfasst weiterhin ein Verfahren zur Reduktion der Milchsäure Ausscheidung im Kot bei Wiederkäuern, dadurch gekennzeichnet, dass dem Wiederkäuer eine stärkereiche Ration mit einem Stärkeanteil von mindestens 25 Gew.% Stärke bezogen auf die Gesamtfuttertrockenmasse verfüttert wird und dem Wiederkäuer eine pansenstabile exogene alpha-Amylase zugeführt wird.

In einer besonderen Ausführungsform beträgt der Stärkeanteil bezogen auf die Gesamtfuttertrockenmasse mindestens 30, 35, 40, 45, 50, 55, 60, 65, 70, 75 oder 80 Gew.%, je nachdem, um welche Wiederkäuer es sich handelt und in welchem Produktions- oder Laktationsabschnitt sich die Tiere befinden.

Unter pansengeschützter Amylase im Sinne der Erfindung ist eine derart formulierte Amylase gemeint, die eine verzögerte Freisetzung der Amylase, vorzugsweise durch den sauren pH im Labmagen, aus der Formulierung erst nach Passage des Pansens aufweist. Vorzugsweise beträgt die maximale Freisetzung nach 6h unter den Pansenbedingungen 25% des Enzyms. Vorzugsweise werden 50-100% der anfänglich vorhandenen Amylase innerhalb von 6-24h im an den Pansen anschliessenden Verdauungstrakt freigesetzt.

Es ist auch denkbar, dass eine alpha-Amylase eingesetzt wird, die durch molekularbiologische Verfahren so verändert wird, dass sie nicht mehr durch die im Pansen vorhanden Mikroorganismen abgebaut werden kann.

Vorzugsweise ist die pansengeschützte Amylase pelletierstabil, entweder durch die Eigenschaften des Proteins selbst oder durch die Art der Formulierung, d.h. sie erleidet keine signifikanten Aktivitätsverluste unter Pelletierbedingungen wie heissem Dampf von 70 bis 100 °C, Druck und Scherkräften, wie sie bei der Herstellung von Futtermittelpellets auftreten. Dies ermöglicht die Einarbeitung der pansengeschützten alpha-Amylase in übliche Futtermittelpellets und damit eine leichtere Dosierung im Stall bei der Verfütterung.

Die Menge an Stärke, die ausgehend vom Stärkeanteil im Futtermittel den Pansen passiert und im Darm zur Milchsäure umgesetzt werden kann, variiert je nach Art des Getreides, in dem die Stärke vorhanden ist, da manche Getreidestärken leichter im Pansen von den Mikroorganismen abbaubar sind, andere aufgrund von sekundären Getreidebestandteilen, wie bspw. des Zeins im Fall von Mais, die einen natürlichen Pansenschutz darstellen, deutlich schwerer abbaubar sind. So wird im Fall von Mais die darin enthaltene und verfütterte Stärke nur zu 50% im Pansen abgebaut, sodass von 2 kg verfütterter Stärke noch 1 kg den Pansen passiert, im Fall von Gerste wird die verfütterte Stärke zu 75 %, im Fall von Weizen zu 85% im Pansen abgebaut, sodass von 4kg bzw. 6,7 kg verfütterter Stärke nur noch 1 kg nach dem Pansen vorhanden sind. Entsprechend ist die Menge, d.h. Units, an Amylase, die dem Futter zugesetzt wird abhängig von der Art des Getreides bzw. der Getreidestärke. Unter Stärke im Futter wird vorliegend der Anteil an Stärke im verfütterten Getreide oder der Silage verstanden, die an die Tiere verfüttert werden.

In einer Ausführungsform werden daher mindestens 26000, vorzugsweise mindestens 30000, insbesondere mindestens 40000, Units Amylase pro kg nach dem Pansen verfügbare Stärke eingesetzt.

In einer anderen Ausführungsform werden mindestens 4000, vorzugsweise mindestens 6500, insbesondere mindestens 13000, Units Amylase pro kg Stärke im Futter eingesetzt, je nach Art der Stärke und deren Abbaugrad im Pansen.

Vorzugsweise werden im Fall von Weizen bzw. auf Weizen basierendem Futter mindestens 4000, vorzugsweise mindestens 5000, insbesondere mindestens 6000, Units Amylase pro kg Stärke im Futter eingesetzt.

Vorzugsweise werden im Fall von Gerste bzw. auf Gerste basierendem Futter mindestens 6500, vorzugsweise mindestens 8000, insbesondere mindestens10000, Units Amylase pro kg Stärke im Futter eingesetzt.

Vorzugsweise werden im Fall von Mais bzw. auf Mais basierendem Futter mindestens 13000, vorzugsweise mindestens 17000, insbesondere mindestens 20000, Units Amylase pro kg Stärke im Futter eingesetzt.

Im Fall von selten vorkommenden aber denkbaren gemischten Getreiderationen, werden die entsprechenden Getreidebestandteile und deren Stärkeanteile entsprechend anteilsmässig bei der Berechnung der mindestens einzusetzen Menge an Amylase berücksichtigt.

Vorteilhafterweise wird durch das erfindungsgemässe Verfahren die mittlere Milchsäure Ausscheidung im Kot pro Tag gegenüber der gleichen stärkereichen Ration ohne eine exogene alpha-Amylase um mindestens 40%, vorzugsweise mind. 50%, insbesondere mind. 60, 70, 80 oder 90 %, gesenkt. Damit wird eine Absenkung des pH im Dickdarm und Entzündung der Darmschleimhaut vermieden bzw. das Risiko der Entzündung deutlich gesenkt. Die Höhe der Absenkung ist hierbei abhängig vom Stärkeanteil in der Ration. So ist bei höheren Stärkeanteilen eine entsprechend höhere Absenkung notwendig, um die absolute Milchsäuremenge, die pro Tag produziert und ausgeschieden wird, auf einem Darm- bzw. Darmschleimhaut verträglichen Niveau zu halten. Unter der mittleren Milchsäureausscheidung pro Tag wird gemäss der vorliegenden Erfindung der Mittelwert der täglichen Ausscheidungen im Kot aus 5 aufeinanderfolgenden Tagen verstanden.

Vorzugsweise sind die Wiederkäuer ausgewählt aus der Gruppe bestehend aus Rindern, insbesondere Milchkühen oder Mastrindern, Schafen und Ziegen.

### Figurenbeschreibung

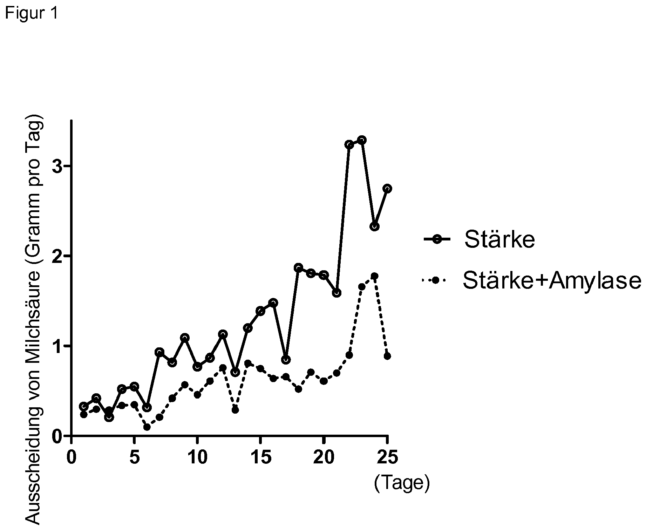

Figur 1 zeigt die tägliche Ausscheidung von Milchsäure im Kot von Färsen, die über eine abosomale Infusion von Maisstärke mit/ohne exogener alpha-Amylase versorgt wurden. Tägliche Dosierung von Stärke: 0.95 kg (Tag 1-5), 1.21 kg Tag (6-10), 1.42 kg (Tag 11-15), 1.73 kg (Tag 16-20), und 1.99 kg(Tag 21-25), ohne (-) und mit (···) Amylase.

### Beispiele

Vier pansenfistulierte Färsen wurden in einem Lateinischen Quadrat verwendet (2×2). Die Färsen erhielten täglich auf zwei gleiche Mahlzeiten aufgeteilt, 5,5 kg einer stärkefreien Ration, die aus 64,9% Wiesenheu, 33% Trockenschnitzeln, 0,9% Harnstoff und 1,2% eines Mineralfutters bestanden (Basis: TS).

Der Versuch bestand aus 2 Perioden von jeweils 35 Tagen wobei einer 10 Tage Anpassung eine 25 tägige Stärkeinfusions- (10 h/Tag) und Sammelperiode folgte. Für die Infusionen wurde eine native Mais-Stärke verwendet (97% Stärke-Gehalt in TS-Basis, Maisita 21.000, Agrana AG, Wien, Austria).

Die Färsen wurden zunächst an die Stärkeverdauung durch eine Stärkeinfusion in den Labmagen gewöhnt. Dabei bestand die Gewöhnung in einer langsam steigenden Infusion von Stärke in den Labmagen. So wurden ab Tag 5 vor der Sammelperiode 0,2 bis 1 kg Stärke pro Tag infundiert. Während der folgenden 30 Infusionstage wurden Stärkemengen von 0,95; 1,21; 1,42; 1,73 und 1,99 kg/Tag, über eine Suspension von 1 kg Stärke pro 10 Liter Wasser appliziert. Diese Abstufungen wurden jeweils 5 Tage durchgehalten. Die Infusionen mit Stärke wurden jeweils mit oder ohne zugesetzter exogener Amylase verabreicht. Die Amylase war zuvor mit destilliertem Wasser in Konzentrationen von 4, 5, 6, 7 oder 8% (mit der Stärkeinfusion ansteigend) eingestellt worden. Diese Mischung wurde mit einer Rate von 50 ml/h infundiert. So kamen 2 bis 4 ml des Enzyms täglich zum Einsatz. Als alpha-Amylase wurde Distizym BA-N von Erbslöh verwendet (13.243 U/ml, gemessen bei pH 7,5). Die Amylase Aktivität wurde bestimmt durch messen der Freisetzung von reduzierenden Zuckerenden von einem Stärkesubstrat bei pH 7,5 und 37 °C. Dafür wurde das Enzym mit 1% gelöster Maisstärke für 10 Minuten bei 37 °C inkubiert. Der pH wurde unter Verwendung eines Tris-Puffers konstant bei pH 7,5 gehalten und gleichzeitig wurden 2mM Magnesiumchlorid sublementiert. Die freigesetzten Zuckerreste wurden mittels BCA (Bichinchoninic) Assay bei einer Wellenlänge von 560 nm bestimmt. Die Enzym Units wurden mittels einer Kalibrierung mit Maltose bestimmt und 1 Unit entspricht vorliegend 1 µmol freigesetzte reduzierende Zuckerenden pro Minute. Somit sind zwischen ca. 26.000 und 52.000 U pro Tier und Tag appliziert worden.

Titandioxyd (10 g/Tag) wurde ruminal in zwei gleichen Gaben täglich zur Schätzung der Kot Menge verabreicht. Vier rektale Kotproben pro Tag wurden während der Sammelprobe gezogen, pro Tier gemischt und zuletzt auf TS und Milchsäuregehalt untersucht. Die Milchsäurekonzentration wurde in Form der D- und L-Milchsäure mittels eines Testkits gemessen (Lactate-UV Test Kit; Boehringer Ingelheim Pharma GmbH & Co. KG, Mannheim, Deutschland).

Die abosomale Infusion von Stärke in zunehmenden Mengen, führte zu einer linearen Steigerung der Milchsäureausscheidung über den Kot. Jedoch war die dieser lineare Anstieg ohne die Infusion von Amylase wesentlich stärker. Bei gleichzeitiger Infusion von Amylase war die Laktatausscheidung über Kot deutlich gebremst. Dies wurde besonders während der letzten Versuchstagen deutlich, als hohe Stärkemengen zum Einsatz kamen. So stieg die Laktatausscheidung mit bzw. ohne Amylase, von 0,29 g/Tag an Tag 1 auf 0,89 g/Tag bzw. auf 2,75 g/Tag an Tag 25, an (Figur. 1).
BCA (Bichinchoninic) Assay - Quantifizierung von reduzierenden Enden nach Amylase-Verdau von Stärke

### Lösungen:

### BCA Reagenz A

64 mg/mL sodium carbonate monohydrate (124g/mol → 0,516M)
24 mg/mL sodium bicarbonate (84,01g/mol → 0,286M)
in gewünschtem Volumen VE-Wasser lösen,
1.95 mg/mL BCA (388,28g/mol → 5mM)
Sterilfiltrieren und bei 4°C lagern.

### BCA Reagenz B

1.24 mg/mL cupric sulfate pentahydrate (249,69g/mol → 5mM)
1.26 mg/mL L-serine (105,09g/mol → 12mM)
in VE-Wasser lösen,
Sterilfiltrieren und bei 4°C lagern.

| Acetatpuffer | Tris-Puffer |
|---|---|
| 200mM Natriumacetat | 50mM Tris-HCl Puffer |
| 2mM MgCl2*6H2O | 2mM MgCl2 |
| pH 4,5 | pH 7.5 |

### 2% Stärke Lösung

2g Mais-Stärke in 100mL VE-Wasser geben,
Lösung in ein kochendes Wasserbad geben und rühren bis alles gut gelöst ist.
Rühren nicht abschalten, damit sich nichts absetzen kann.

### 10mM Maltose Lösung (Standard)

### Enzymlösungen:

Distizym BA-N vom Juli 2014
Distizym BA-N vom März 2015

### Enzymverdünnungen:

1:4.000 - 1:6.000 - 1:9.000 - 1:13.500 - 1:20.250
in Acetatpuffer bzw. Tris-Puffer

### Durchführung:

### Maltosestandard:

| Konz. im Test (µM) | Vol. 10 mM Maltose in µL | Vol. VE-Wasser (µL) | µmol |
|---|---|---|---|
| 0 | 0 | 1000 | 0 |
| 100 | 10 | 990 | 0.001 |
| 200 | 20 | 980 | 0.002 |
| 300 | 30 | 970 | 0.003 |
| 400 | 40 | 960 | 0.004 |
| 500 | 50 | 950 | 0.005 |
| 600 | 60 | 940 | 0.006 |

Standard kann bei 4°C gelagert werden.

Aus einer Stammlösung werden die oben angegebenen Konzentrationen in Doppelbestimmung angesetzt.

Je Konzentration werden 10µL zur Nachweisreaktion
hinzugegeben.

### Reaktionsansatz - Enzymreaktion:

| | Volume (µL) |
|---|---|
| 2% Stärke | 125.0 |
| 200mM Ac pH 4,5 bzw. Tris-Puffer pH 7,5 | 62.5 |
| VE-Wasser | 52.5 |
| Enzym-Lösung | 10.0 |
| Gesamtvolumen: | 250.0 |

Inkubation für 10 min
bei 37°C

### Reaktionsansatz - Nachweisreaktion:

BCA Reagenzien A und B im Verhältnis 1:1
mischen,
100µL der Mischung in ein PRC-Reaktionsgefäß geben,
10µL Probe (Standard/Kontrolle/Enzymreaktion) hinzugeben,
auf Eis stehen lassen, bis alle Proben gesammelt sind,
35min bei 80 °C inkubieren (PCR-Cycler),
10min bei 25°C inkubieren,
10min bei RT inkubieren,
100µL der Lösung in eine Flat-Bottom MTP
Überführen,
Endpunktmessung bei 560nm.

## Patentansprüche

1. Verwendung einer pansengeschützten exogenen alpha-Amylase zur Senkung der Milchsäure Ausscheidung im Kot bei Wiederkäuern, welche mit stärkereichen Rationen mit einem Stärkeanteil von mindestens 25 Gew.% bezogen auf die Gesamtfuttertrockenmasse gefüttert werden.

2. Verwendung nach Anspruch 1, **dadurch gekennzeichnet, dass** der Stärkeanteil mindestens 30, 35, 40, 45, 50, 55, 60, 65, 70, 75 oder 80 Gew. % beträgt

3. Verwendung nach einem der Ansprüche 1 oder 2, **dadurch gekennzeichnet, dass** mindestens 26000 Unit, vorzugsweise mindestens 30000 Units, insbesondere mindestens 40000 Units, Amylase pro kg nach dem Pansen verfügbare Stärke eingesetzt werden.

4. Verwendung nach einem der vorhergehenden Ansprüche, **dadurch gekennzeichnet, dass** mindestens 4000 Unit, vorzugsweise mindestens 6500 Units, insbesondere mindestens 13000 Units, Amylase pro kg Stärke im Futter, eingesetzt werden.

5. Verwendung nach einem der vorhergehenden Ansprüche, **dadurch gekennzeichnet, dass** die mittlere Milchsäure Ausscheidung im Kot pro Tag gegenüber der gleichen stärkereichen Ration ohne exogene alpha- Amylase um mindestens 40 %, vorzugsweise mind. 50%, insbesondere mind. 60, 70, 80 oder 90 %, gesenkt wird.

6. Verwendung nach einem der vorhergehenden Ansprüche, **dadurch gekennzeichnet, dass** die Wiederkäuer ausgewählt sind aus der Gruppe bestehend aus Rindern, insbesondere Milchkühen oder Mastrindern, Schafen und Ziegen.

7. Verwendung einer pansengeschützten exogenen alpha-Amylase zur Erhöhung der post-Pansen Stärkeverwertung bei Wiederkäuern, welche mit stärkereichen Rationen mit einem Stärkeanteil von mindestens 25 Gew.% bezogen auf die Gesamtfuttertrockenmasse gefüttert werden.

8. Bereitstellung einer pansengeschützen exogenen alpha-Amylase zur Behandlung einer stärkebedingten Dickdarm Acidose bei Wiederkäuern.

9. Verfahren zur Reduktion der Milchsäure Ausscheidung im Kot bei Wiederkäuern, **dadurch gekennzeichnet, dass** dem Wiederkäuer eine stärkereiche Ration mit einem Stärkeanteil von mindestens 25 Gew.% Stärke bezogen auf die Gesamtfuttertrockenmasse verfüttert wird und dem Wiederkäuer eine pansenstabile exogene alpha-Amylase zugeführt wird.

10. Verfahren nach Anspruch 9, **dadurch gekennzeichnet, dass** der Stärkeanteil mindestens 30, 35, 40, 45, 50, 55, 60, 65, 70, 75 oder 80 Gew. % beträgt

11. Verfahren nach einem der Ansprüche 9 oder 10, **dadurch gekennzeichnet, dass** mindestens 26000 Unit, vorzugsweise mindestens 30000 Units, insbesondere mindestens 40000 Units, Amylase pro kg nach dem Pansen verfügbare Stärke eingesetzt werden.

12. Verfahren nach einem der Ansprüche 9 bis 11, **dadurch gekennzeichnet, dass** mindestens 4000 Unit, vorzugsweise mindestens 6500 Units, insbesondere mindestens 13000 Units, Amylase pro kg Stärke im Futter, eingesetzt werden.

13. Verfahren nach einem der Ansprüche 9 bis 12, **dadurch gekennzeichnet, dass** die mittlere Milchsäure Ausscheidung im Kot pro Tag gegenüber der gleichen stärkereichen Ration ohne exogene alpha- Amylase um mindestens 40 %, vorzugsweise mind. 50%, insbesondere mind. 60, 70, 80 oder 90 %, gesenkt wird.

14. Verfahren nach einem der Ansprüche 9 bis 13, **dadurch gekennzeichnet, dass** die Wiederkäuer ausgewählt sind aus der Gruppe bestehend aus Rindern, insbesondere Milchkühen oder Mastrindern, Schafen und Ziegen.
